(19) Europäisches Patentamt
European Patent Office
Office européen des brevets

(11) Publication number: **0 641 569 A1**

(12) **EUROPEAN PATENT APPLICATION published in accordance with Art. 158(3) EPC**

(21) Application number: **93905632.1**

(22) Date of filing: **11.03.93**

(86) International application number: **PCT/JP93/00298**

(87) International publication number: **WO 93/17709 (16.09.93 93/22)**

(51) Int. Cl.[6]: **A61K 45/00**, A61K 31/41, C07D 405/04

(30) Priority: **12.03.92 JP 88201/92**

(43) Date of publication of application: **08.03.95 Bulletin 95/10**

(84) Designated Contracting States: **AT BE CH DE DK ES FR GB GR IE IT LI LU MC NL PT SE**

(71) Applicant: **ONO PHARMACEUTICAL CO., LTD.**
**1-5, Doshomachi 2-chome**
**Chuo-ku**
**Osaka 541 (JP)**

(72) Inventor: **SHIKANO, Masahiko**
**619, Nanasaki,**
**Sunami-cho**
**Motosu-gun,**
**Gifu 501-03 (JP)**
Inventor: **NAKAO, Kenzo, Ono Pharmaceutical Co., Ltd.**
**1-5, Doshomachi 2-chome,**
**Chuo-ku**
**Osaka-shi,**
**Osaka 541 (JP)**

(74) Representative: **Bentham, Stephen et al**
**J.A. KEMP & CO.**
**14 South Square**
**Gray's Inn**
**London WC1R 5LX (GB)**

(54) **ANTIPRURITIC.**

(57) An antipruritic containing as the active ingredient a leucotriene $C_4$ or $D_4$ antagonist, in particular, 8-[p-(4-phenylbutoxy)benzoyl]amino-2-(tetrazol-5-yl)-4-oxo-4H-1-benzopyran represented by the formula (I), a harmless salt thereof, or a hydrate thereof. It is useful for treating pruritis, especially, one accompanying hemodialysis.

EP 0 641 569 A1

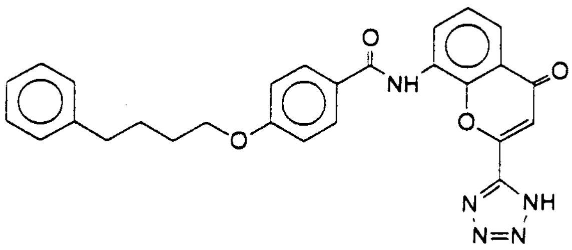

(I)

## Field of the Invention

The present invention relates to treating agents for pruritus cutanea containing, as active ingredient, antagonists of leukotriene $C_4$ or $D_4$ (abbreviated as $LTC_4$ and $LTD_4$, respectively, hereafter)

## Background of the Invention

As patients who continually undergo hemodialysis, increase with the progress of hemodialysis therapy, various complications thereof have become an issue. Especially, one of the complications which the patients have suffered, is pruritus cutanea. Severe itching attacks the patients not only on dialysis but day and night. They have their attention distracted and are unable to get themselves to work at daytime, and they are unable to sleep at night. They often have rash.

Examples of original diseases which lead to hemodialysis therapy include mainly chronic glomerulonephritis, diabetes, and additionally nephrosclerosis, cystic kidney, kidney disorders accompanying various systemic diseases. It is said that pruritus cutanea appears in 80 to 90 % of patients in hemodialysis.

It is considered that pruritus cutanea is induced by various factors, but the mechanism is still unclear.

Until now, various therapies such as administration of antihistamines, antiallergic agents, steroid ointment, radiation of ultraviolet rays, change of hemocatharsis etc., have been carried out, but are not fully efficacious. Further, there is the problem that antihistamines and antiallergic agents induce side effects in digestive system or drowsiness.

On the other hand, $LTC_4$ and $LTD_4$ are physiologically active substances which are released from cell membranes. They were found as a member of the prostaglandin family in 1979. Thereafter, it was found that $LTC_4$ and $LTD_4$ are identical to a substance which had been called slow reacting substance (SRS) or SRS-A up to that time. Therefore, it was considered that $LTC_4$ and $LTD_4$ are important factors which are concerned with appearance of allergic bronchial or pulmonary diseases, allergic shock and various allergic inflammation.

For the purpose of the treatment of such diseases, antagonists against $LTC_4$ and $LTD_4$ were synthesized and many patent applications on various antagonists have been filed until now. However, there is no antagonist for the treatment of pruritus cutanea.

From the above view point, the present inventors have carried out energetic investigation in order to find novel treating agents for pruritus cutanea. As a result, we have found that antagonists against $LTC_4$ or $LTD_4$ are surprisingly effective and have accomplished the present invention.

Until now, the relation between hemodialysis and $LTC_4$ or $LTD_4$ has never been discussed. The present inventors have measured the concentration of leukotrienes in blood of patients in hemodialysis and as a result, it has turned out that the concentration of leukotrienes in blood after hemodialysis unexpectedly and significantly increases as compared with the concentration before hemodialysis (see Reference Example 1 hereinafter described). We have considered that the increase in concentration of leukotrienes is a main factor in pruritus cutanea induced by hemodialysis.

Remarkable improvement in itching has induced by the administration of 8-[p-(4-phenylbutoxy)benzoyl]-amino-2-(tetrazol-5-yl)-4-oxo-4H-1-benzopyran semihydrate, which is a strong antagonist against $LTC_4$ and $LTD_4$, to patients in pruritus.

Accordingly, it has been found that broad compounds having antagonistic activity against $LTC_4$ or $LTD_4$, not limited to the above compound, are very useful for the treatment of pruritus cutanea induced by the increase in the concentration of $LTC_4$ or $LTD_4$, represented by hemodialysis.

## Disclosure of the Invention

The present invention relates to treating agents for pruritus cutanea containing, as active ingredient, antagonists of $LTC_4$ or $LTD_4$.

The antagonists of $LTC_4$ or $LTD_4$, used in the present invention include not only all compounds which are already known by the publication of patent applications, the publication of literatures and the publication in symposium and meetings, but also antagonists against $LTC_4$ and $LTD_4$ which will be newly prepared in the future. Furthermore, it includes compounds represented as SRS antagonists and SRS-A antagonists.

Examples of the representative compounds are:

(1) RG-12525: 2-[[4-[[2-(1H-tetrazol-5-ylmethyl)phenyl]methoxy]phenoxy]methyl]quinoline,

(2) sulukast: (1S, 2R)-5-[3-[2-(2-carboxyethylthio)-1-hydroxypentadeca-3(E), 5(Z)-dienyl]phenyl]-1H-tetrazole,

(3) DS-4574: 6-(2-cyclohexylethyl-1,3,4-thiaziazol[3,2-a]-1,2,3-triazol[4,5-d]pyrimidine-9(1H)-one,

(4) Dup-654: 2-benzyl-1-naphthol,

(5) MK-571: 5-[3-[2-(7-chloroquinolin-2-yl)vinyl]phenyl]-8-(dimethylcarbamoyl)-4,6-dithiaoctanoic acid,

(6) AS-35: 9-(4-acetyl-3-hydroxy-2-propylphenoxymethyl)-3-(1H-tetrazol-5-yl)-4-pyrid[1,2-a]pyrimidin-4-one,

(7) SC-45662: [2-[3,5-bis(tert-butyl)-4-hydroxyphenylthio]-1-methylpropoxy]acetic acid,

(8) ICI-204219: N-[4-[5-(cyclopentyloxycarbonylamino)-1-methylindol-3-ylmethyl]-3-methoxybenzoyl]-2-methylbenzenesulfonamide,

(9) SK&F-104353: 2(S)-hydroxy-3(R)-(2-carboxyethylthio)-3-[2-(8-phenyloctyl)pheny]propionic acid,

(10) pranlukast: 8-[p-(4-phenylbutoxy)benzoyl]amino-2-(tetrazol-5-yl)-4-oxo-4H-1-benzopyran

or non-toxic salts thereof, or hydrates thereof; and 8-[p-(4-phenylbutoxy)benzoyl]amino-2-(tetrazol-5-yl)-4-oxo-4H-1-benzopyran and non-toxic salts thereof, and hydrates thereof, of the formula:

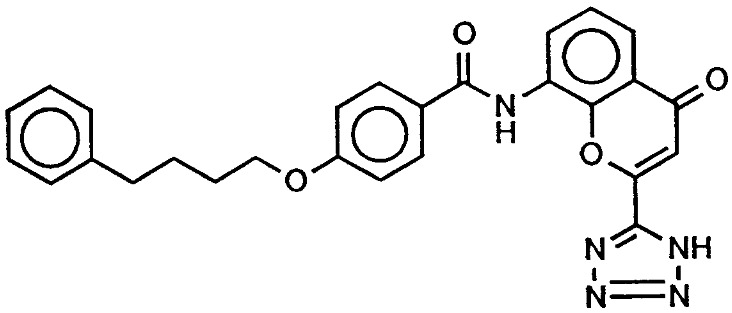

(I)

are especially preferred.

The compound of the formula (I) and the process for the preparation thereof are disclosed in detail in the specification of the Japanese Patent Kokai No. 61-50977.

The antagonists of $LTC_4$ or $LTD_4$, used in the present invention can be used as excellent treating agents for pruritus cutanea. The term of "pruritus cutanea" herein include any cases which induce itching, for example pruritus cutanea induced by hemodialysis. Further, it is confirmed that the antagonists of $LTC_4$ or $LTD_4$, used in the present invention have extremely low toxicity.

For the purpose above described, the antagonists of $LTC_4$ or $LTD_4$ may be normally administered systemically or partially, usually by oral or parenteral administration.

The doses to be administered are determined depending upon age, body weight, symptom, the desired therapeutic effect, the route of administration, and the duration of the treatment etc. In the human adult, the doses per person are generally between 10 mg and 1000 mg, by oral administration, up to several times per day, and between 100 $\mu$g and 10 mg, by parenteral administration (preferably intravenous or intracutaneous administration), up to several times per day.

As mentioned above, the doses to be used depend upon various conditions. Therefore, there are cases in which doses lower than or greater than the ranges specified above may be used.

When the compounds of the present invention are administered, they are used as solid compositions, liquid compositions or other compositions for oral administration, or as injections, liniments or suppositories etc. for parenteral administration.

Solid compositions for oral administration include compressed tablets, pills, capsules, dispersible powders, and granules.

In such compositions, one or more of the active compound(s) is or are admixed with at least one inert diluent (lactose, mannitol, glucose, hydroxypropyl cellulose, microcrystalline cellulose, starch, polyvinylpyrrolidone, magnesium metasilicate aluminate, etc.).

The compositions may also comprise, as is normal practice, additional substances other than inert diluents: e.g. lubricating agents (magnesium stearate etc.), disintegrating agents (cellulose calcium glycolate, etc.), assisting agents for dissolving (arginine, glutamic acid, aspartic acid etc.), stabilizing agents (human serum albumin, lactose etc.) and the other additional substances [PEG, PVP, HPC, surface active agents (HCO, Tween (registered trade mark)) etc.].

The tablets or pills may, if desired, be coated with a film of gastric or enteric material (sugar, gelatin, hydroxypropyl cellulose or hydroxypropylmethyl cellulose phthalate, etc.).

Capsules include hard capsules and soft capsules.

Liquid compositions for oral administration include solutions, emulsions, suspensions, syrups and elixirs. In such compositions, inert diluent(s) commonly used in the art (purified water, ethanol etc.) are included.

Besides inert diluents, such compositions may also comprise adjuvants such as wetting agents and suspending agents, sweetening agents, flavoring agents, perfuming agents, and preserving agents.

Other compositions for oral administration comprise one or more of the active compound(s) and include spray compositions which may be prepared by known methods. Spray compositions may comprise additional substances other than inert diluents: e.g. stabilizing agents (sodium sulfate etc.), isotonic buffer (sodium chloride, sodium citrate, citric acid, etc.). For preparation of such spray compositions, for example, the method described in the United States Patent No. 2,868,691 or 3,095,355 may be used.

Injections for parenteral administration include sterile aqueous or non-aqueous solutions, suspensions and emulsions. In such an injections, one or more of the active compound(s) is or are admixed with at least one inert aqueous diluent (distilled water for injection and physiological salt solution) or inert non-aqueous diluent (propylene glycol, polyethylene glycol, olive oil, ethanol, POLYSORBATE80 (registered trade mark), etc.).

Injections may comprise additional diluents: e.g. preserving agents, wetting agents, emulsifying agents, dispersing agents, stabilizing agent (human serum albumin, lactose), assisting agents for dissolving (arginine, glutamic acid, aspartic acid, polyvinylpyrrolidone etc.).

They may be sterilized for example, by filtration (bacteria-retaining filter etc.), by incorporation of sterilizing agents in the compositions or by irradiation or after carrying out such a procedure, they may be used in the form of solid compositions by the method of freeze drying etc. and which may be dissolved in sterile water or some other sterile diluent(s) for injection immediately before used.

Best Mode of the Invention

The following reference examples and examples illustrate the present invention, but not limit the present invention.

Reference Example 1:

Change of the Concentration of Leukotrienes in Plasma in Patients of Hemodialysis

Blood was collected from 40 patients of hemodialysis before dialysis, and after 15 minutes from dialysis (referred to "after dialysis" hereafter), respectively. The concentration of leukotrienes ($LTC_4$, $LTD_4$, $LTE_4$ and $LTF_4$) in plasma was measured and then the average value thereof was calculated. The results were shown in Table 1. $LTE_4$ is a metabolite of $LTD_4$ and $LTF_4$ is a metabolite of $LTE_4$.

Table 1

| | Concentration of leukotrienes (ng/ml) |
|---|---|
| Before Dialysis | 0.15 ± 0.04 |
| After Dialysis | 0.17 ± 0.04 |

It has been turned out that the concentration after dialysis is higher than that before dialysis significantly ($p < 0.05$). Therefore, it is considered that the concentration relates to pruritus cutanea in hemodialysis.

Example 1:

Treatment for Pruritus Cutanea in Hemodialysis

8-[p-(4-phenylbutoxy)benzoyl]amino-2-(tetrazol-5-yl)-4-oxo-4H-1-benzopyran•1/2 hydrate (abbreviated as "Compound A" hereafter) were administered at the dose of 225 mg or 450 mg per day to four patients in hemodialysis (dialyzed for 3 to 11 years) who have severe itching and show no effect with other agents [no effect even with antihistamine, azerastin (anti-allergy agent already on the market) and steroid ointment]. The evaluation was carried out by improvement score of subjective symptom according to "The Standard of degree of Itching" and of rash (judged by slide). The results were shown in Table 2. And, the standard of degree of itching was shown in Table 3.

Table 2: Treating Effect on Pruritus Cutanea in Hemodialysis

| Patients | Degree of Itching (Score) | | | | | | | | Degree of Rash (Judged by slide) (After 8 weeks) | Side Effect |
|---|---|---|---|---|---|---|---|---|---|---|
| | Daytime | | | | Nighttime | | | | | |
| | Before Admini-stration | After 2 weeks | After 4 weeks | After 8 weeks | Before Admini-stration | After 2 weeks | After 4 weeks | After 8 weeks | | |
| A (female) | 4 | 3 | 3 | 2 | 4 | 3 | 3 | 2 | improved | nothing |
| B (male) | 4 | 3 | 2 | 2 | 3 | 2 | 2 | 2 | improved | nothing |
| C (male) | 4 | 4 | 3 | 2 | 3 | 2 | 2 | 1 | significantly improved | nothing |
| D (male) | 4 | 4 | 3 | 3 | 4 | 4 | 3 | 3 | failed to judge because rash before administn is small | nothing |

Table 3

| Standard of degree of Itching | | |
|---|---|---|
| Score | Symptom (Daytime) | Symptom (nighttime) |
| **4** (severe itching) | He can not bear the itching any longer. The itching can not be abated even by scratching his affected part, and rather become severe more and more. He can not work or study because of itching. | He can hardly sleep because of itching. He scratches at all times while sleeping. Once he scratches, the itching become severe more and more. |
| **3** (moderate itching) | He has strong itching and scratches even in the presence of others. He becomes irritated because of itching and always scratches. | The itching awakes him. After scratching, he can sleep. He scratches unconsciously while sleeping. |
| **2** (light itching) | The itching can abate after lightly scratching. He does not care the itching. | The itching can abate after lightly scratching. The itching does not awake him. |
| **1** (slight itching) | Sometimes he has itching, but can bear it without scratching. | When he goes to bed, he has itching slightly, but does not scratch consciously. He can sleep well. |
| **0** (no symptom) | He has no itching or almost no itching. | He has no itching or almost no itching. |
| by Akira Shiratori (Sapporo City Hospital) | | |

Table 2 shows:

(1) Any patients to whom the administration of agents other than Compound A exerted no effect, became better by administration of Compound A.

(2)Improvement was shown not only in subjective symptom but objective symptom, and further at daytime and nighttime.

(3) Any patients had no side effect.

Therefore, it was confirmed that Compound A is very useful as treating agent of pruritus cutanea.

Example 2:

Acute Toxicity

Compound A was orally or subcutaneously administered to each ten male and female 6-weeks aged Jcl-ICR mice and Jcl-SD rats, respectively per group. Two groups of Compound A (1000 and 2000 mg/kg) were used by suspending into 0.5% carboxymethyl cellulose (CMC) aqueous solution at the concentration of 100 mg/ml. The results were shown in Table 4.

Table 4

| Acute Toxicity | | | |
|---|---|---|---|
| Species of Animal | Route of Administrarion | $LD_{50}$ (mg/kg) | |
| | | male | female |
| Mice | orally<br>subcutaneously | > 2000<br>> 2000 | > 2000<br>> 2000 |
| Rats | orally<br>subcutaneously | > 2000<br>> 2000 | > 2000<br>> 2000 |

As shown in Table 4 above, it was confirmed that the toxicity of Compound A is very low in oral and subcutaneous administration and it can be used quite safely as pharmaceutical agent.

Example of Pharmaceutical Composition

Following components were admixed in conventional manner, and then placed into gelatin capsules to obtain 100 capsules.

| | |
|---|---|
| Compound A | 112.5 mg |
| lactose | 52.5 mg |
| magnesium stearate | proper amounts |
| hydroxypropyl cellulose | proper amounts |

## Claims

**1.** A treating agent for pruritus cutanea containing, as active ingredient, an antagonist of leukotriene $C_4$ or $D_4$.

**2.** A treating agent according to claim 1, wherein the antagonist of leukotriene $C_4$ or $D_4$ is 8-[p-(4-phenylbutoxy)benzoyl]amino-2-(tetrazol-5-yl)-4-oxo-4H-1-benzopyran,
or a non-toxic salt thereof, or a hydrate thereof.

**3.** A treating agent according to claim 1 or 2, wherein pruritus cutanea is that induced by hemodialysis.

# INTERNATIONAL SEARCH REPORT

International application No. PCT/JP93/00298

**A. CLASSIFICATION OF SUBJECT MATTER**

Int. $Cl^5$ A61K45/00, A61K31/41, C07D405/04

According to International Patent Classification (IPC) or to both national classification and IPC

**B. FIELDS SEARCHED**

Minimum documentation searched (classification system followed by classification symbols)

Int. $Cl^5$ A61K45/00, A61K31/41, C07D405/04

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| A | JP, A, 1-31757 (Lee Roy Morgan), February 2, 1989 (02. 02. 89), & EP, A, 300100 | 1-3 |
| A | WO, A, 9204311 (Schering A.G.), March 19, 1992 (19. 03. 92), & DE, A, 4028866 | 1-3 |

☐ Further documents are listed in the continuation of Box C. ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| May 10, 1993 (10. 05. 93) | June 1, 1993 (01. 06. 93) |
| Name and mailing address of the ISA/ Japanese Patent Office | Authorized officer |
| Facsimile No. | Telephone No. |